# EUROPEAN PATENT APPLICATION

(11) **EP 3 597 104 A1**
(43) Date of publication of application: **22.01.2020**
(21) Application number: 18184355.8
(22) Date of filing: 19.07.2018
(51) Int. Cl.: A61B 6/00, G06F 3/01, A61B 6/03, A61B 6/04, A61B 5/055

(54) **GESTURAL SCAN PARAMETER SETTING**

(71) Applicant: Koninklijke Philips N.V., 5656 AE Eindhoven (NL)
(72) Inventor: SCHRAVEN, Eckhardt Henricus Matheus, 5656 AE Eindhoven (NL); TAN, Wee Kar, 5656 AE Eindhoven (NL); FEUERABEND, Andrea, 5656 AE Eindhoven (NL); VERSTEEG, Dennis John, 5656 AE Eindhoven (NL)
(74) Representative: de Haan, Poul Erik

(57) **Abstract**

The present invention relates to a device in which a scan parameter for the medical imaging device is determined based on positional and gestural information of an operator of the medical imaging device.

## Description

### FIELD OF THE INVENTION

The present invention generally relates to a device, method and computer program product to set medical scan parameters, as well as a medical imaging device.

### BACKGROUND OF THE INVENTION

In medical imaging, such as diagnostic and interventional X-ray imaging (DXR, IXR), computed tomography (CT), magnetic resonance imaging (MRI), positron emission tomography (PET), single-photon emission computed tomography (SPECT) and the like, many efforts have been made to reduce subject discomfort and increase subject safety through improved workflow and procedures. These also increase the efficiency of use of the device and subject throughput which allows for an increased amount of subjects to be scanned, thereby reducing subject waiting times and increasing potential hospital revenues.

One part of the workflow in all medical scanning devices includes scan planning in which scan parameters need to be determined and set for the scan. Such scan parameters include for instance a scan start and end position (defining a scan range); scan speed; minimum and maximum scan dose for radiation-type medical imaging; and/or object location, such as an organ of interest location or a metallic object location.

Such scan parameters are highly dependent on the subject and/or the procedure, for instance scan parameter settings are very different for a knee scan, lung scan, cardiac scan or brain scan; for diagnostic or interventional procedures; a child or adult subject; a male or female subject; an obese or thin subject; position on the subject support, etc. Because of this it is necessary to set the scan parameters for each procedure with limited opportunities to use standardized settings.

For radiation based medical imaging, such as DXR, IXR and particularly CT, this procedure is crucial, since x-ray radiation may cause harm to the subjects (and in some cases the medical staff) and only the area of interest must be irradiated. To achieve this a so-called scout scan or pre-scan is normally performed in which the subject is scanned with a low-dose radiation beam to determine position of the subject and any objects-of-interest (such as organs). Once these are known the scan area is determined and programmed into the medical imager for the full scan. In the meantime the subject must remain completely immobile during the pre-scan and the time it takes for an operator (such as a physician or radiation technician) to determine the scan parameters and input them into the console, which is usually away from the medical imaging device (often behind a wall or in a separate room to avoid radiation exposure to the operator) and during the time of the actual full-dose scan. This is uncomfortable for the subject and may not even be effectively possible, causing misalignments. Also some subjects may be physically or mentally too unstable to hold the same position for too long. Furthermore, this is very time consuming, reducing the throughput times of the device. Subjects need to be informed and instructed on how to position themselves and hold still for a certain amount of time, which is not always possible or is easily forgotten due to stress, especially when a procedure takes a long time.

In trauma situations it is even more important to perform a scan as fast as possible, since often every minute, or even every second, counts in such situations. In such an occasion radiation dose and image quality is still an issue, but is usually considered less important, so scan parameter settings may be set much quicker. Still it involves positioning a subject, then relocating to the console and enter the parameters there. This is valuable time that is preferably rather used to treat the subject. Due to stress in the subject or situation there may be more chance of voluntary or involuntary movements in trauma situations as well that may disturb the scan itself and the usability of the results, causing inaccurate diagnoses or the need for time-consuming rescans.

Because of the above it is very desirable to be able to reduce the time of scan parameter setting of a medical imaging device.

### SUMMARY OF THE INVENTION

The presently claimed invention, amongst others, intends to provide a system, method and software to set medical scan parameters that achieve the above-mentioned desirable improvements.

Embodiments according to the present invention are directed to a medical scan parameter setting device for a medical imaging device for scanning a subject by an operator controlling the medical imaging device. The medical scan parameter setting device comprises a camera arrangement that comprises at least one camera to obtain positional information of at least part of the operator and a gesture detector configured to detect gestural information from the operator. The medical scan parameter setting device further comprises a parameter provider configured to provide a medical scan parameter setting based on said positional information and said gestural information.

In other words, the operator uses gestures that are detected, together with positional information, by the medical scan parameter setting device to set the scan parameters. Such a medical scan parameter setting device is able to provide scan parameters in a much faster manner, since the operator does not need to relocate to a work station and can stay by the subject's side and also no scout scan is necessary, thereby also reducing radiation dose received by the subject and less change of misalignments due to voluntary or involuntary movements during the whole pre-scan procedure.

In an embodiment the positional information includes a position of at least one hand of the operator; and, preferably, a position of at least part of the subject and/or of a subject support for supporting the subject during a medical scan with the medical imaging device; and, more preferably, a relative position of the at least one hand of the operator in relation to the position of at least part of the subject and/or the subject support.

As such the operator may use his hands to perform the gestures to be detected. Since hands are among the most articulate body parts, they are particularly suitable to gesture with. Further when the hand positions of the operator is known with respect to the subject and/or the subject support, then the gestural information may be directly coupled with the subject and more accurate scan setting parameters may be produced.

In an embodiment, the gestural information includes at least one gesture of the at least one hand of the operator, particularly a sequence of gestures of the at least one hand of the operator. Hand gestures, i.e. pre-defined hand movements, may provide more information than immobile hand positions, and may be used to convey more detailed information.

In an embodiment the gesture detector is configured to detect at least one gesture from image data obtained by the at least one camera. As such no additional hardware component is necessary, since the gesture detector may be implemented as a software algorithm or computer program that may be run and stored on a processor.

In an embodiment the medical scan parameter setting device further comprises a gesture comparer that is configured to generate gestural information by comparing the detected at least one gesture to a predefined gesture, preferably from a gesture library, that has a predefined meaning relating to the medical scan, wherein the gestural information comprises the predefined meaning. This is the most efficient way to determine the meaning of a detected hand gesture.

In an embodiment the predefined meaning consists of a meaning selected from the group of start position; end position; scan speed; dose setting; object location, such as an organ of interest location or a metallic object location; subject information, such as subject gender, weight, age; scan start signal; and/or scan abort signal. These are very useful predefined meanings that correspond with scan parameter settings that can be immediately understood and used by the medical imaging device.

In an embodiment the parameter provider is configured to generate the medical scan parameter by combining gestural information and positional information and transmitting said combined information to a medical scan parameter. As such the gestural information, linked to a scan parameter, is linked to a position to allow for positional scan parameters, e.g. scan ranges, to be determined.

In an embodiment the medical scan parameter consists of at least one of a scan parameter selected from the group of: start position; end position; scan speed; minimum scan dose; maximum scan dose; and/or object location, such as an organ of interest location or a metallic object location. These are the most common and useful scan parameters for a medical scan.

In an embodiment the medical scan parameter setting device further comprises a projection device configured to, when in use with a subject present, project information relating to the determined scan parameters on or near the subject, for instance a scan parameter preview, such as a scan range preview; virtual tools, such as scan parameter modification tools; and/or scan control tools. This allows for visualizing the gestured scan parameter settings to determine their usefulness and correctness, and preferably correct them directly, without requiring the operator to leave the side of the subject.

In an embodiment the projection device is further configured to project additional subject data on or near the subject, such as general subject data, such as subject gender, weight, age; anatomical information; and/or subject relevant medical history information, such as previous scan images, processed information from previous scan images, list of medical conditions and/or presence and/or type of implants. This provides the operator with even more information at the side of the subject that may influence him to accept or adapt the provided scan parameter settings.

Further embodiments are directed towards a medical imaging device comprising a medical scan setting device as claimed. Particularly CT scanners and other medical imagers using x-rays or that are used in trauma situations benefit from having a medical scan setting device using gestures.

Further embodiments are directed towards a corresponding method for setting medical scan parameters using gestures and a computer program product equipped to perform the relevant elements of aid method.

Still further aspects and embodiments of the present invention will be appreciated by those of ordinary skill in the art upon reading and understanding the following detailed description. Numerous additional advantages and benefits will become apparent to those of ordinary skill in the art upon reading the following detailed description of preferred embodiments.

### BRIEF DESCRIPTION OF THE DRAWINGS

The present invention is illustrated by drawings of which
Fig. 1 shows a schematic overview of an exemplary medical imager according to the presently claimed invention.
Fig. 2 shows a schematic depiction of a medical imager, a subject and an operator to illustrate the presently claimed invention.
Fig. 3 shows a series of schematic drawings to illustrate various embodiments of scan ranges.
Fig. 4 shows a series of schematic drawings to illustrate various embodiments of adaptable scan ranges.
Fig. 5 shows a series of schematic drawings to illustrate examples of projected control buttons.
Fig. 6 shows a schematic flowchart of a method to set a medical scan parameter according to the presently claimed invention.

The invention may take form in various components and arrangements of components, and in various process operations and arrangements of process operations. The drawings are only for the purpose of illustrating preferred embodiments and are not to be construed as limiting the invention. To better visualize certain features may be omitted or dimensions may be not be according to scale.

### DETAILED DESRIPTION OF EMBODIMENTS

It is one objective of the presently claimed invention to provide a faster device and method to determine medical scan parameters before the start of a medical scan.

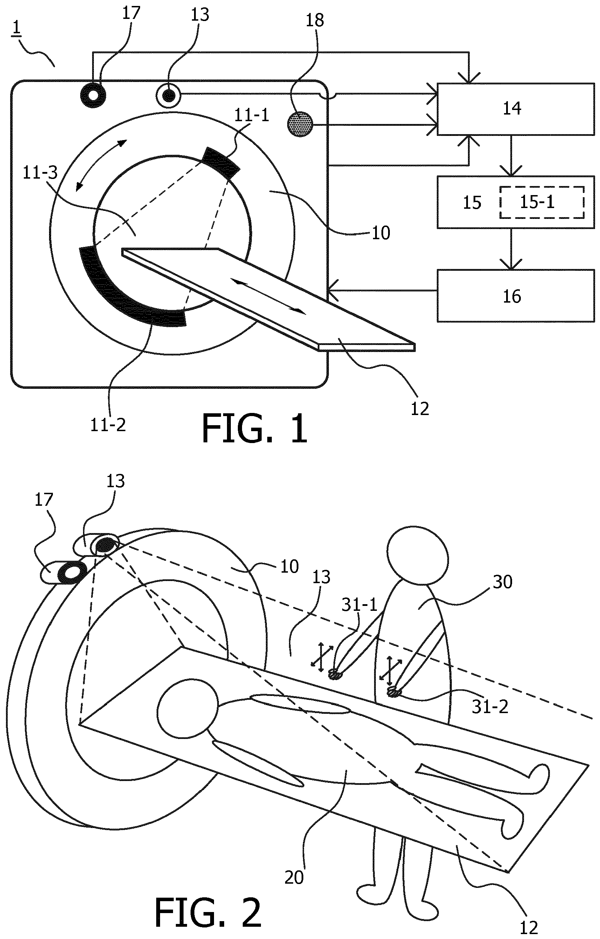

Figure 1 shows an example of an embodiment of a medical scanning device 1 according to the presently claimed invention that achieves said objective.

In this example the medical imaging device 1 is a CT imaging device, but it could be easily adapted for other medical imaging devices (e.g. a DXR, IXR, MR, PET, SPECT or other medical scan) accounting for differences in shape, positioning and the like.

The CT imaging device 1 comprises a rotatable gantry 10 including an x-ray source 11-1 and a radiation detector 11-2 opposite of the source 11-1. When in use the source 11-1 emits x-ray radiation towards the detector 11-2 through examination region 11-3. A subject to be scanned is positioned on a subject support 12, which is moved horizontally through the examination region 11-2 of the rotating gantry 10. As such the subject is imaged three dimensionally.

Since x-ray radiation is harmful to biological tissue it is important to keep the x-ray dose as low as possible and any x-ray exposure restricted to only the part of the subject that is of medical interest. On the other hand a low x-ray dose results in less sharp images. While this may be partly corrected using reconstruction software, still the radiation dose to be administered is high enough to cause damage in a subject. Therefore it is also very important to limit the scan range to cover, preferably, only the tissue of interest to ensure that no malignancy or the like is missed, but also to prevent healthy tissue to be exposed.

To determine the optimal scan parameters such as scan range and radiation dose the most precise available technique is using a scout scan (also known as a pre-scan) in which the subject is scanned with a low dose and a broad scan range. CT scout scans may be performed with a stationary gantry in which a two-dimensional image is obtained. While the exact location of the tissue of interest is obtained, it does expose the subject to additional radiation, albeit at an as low dose as possible. And, at least some time will pass between the scout scan and the actual scan, due to repositioning of the subject support, the operator needing to relocate to the control station and entering the correct scan parameters. In all this time the subject needs to stay exactly in the same position, since any voluntary or involuntary movement results in a difference between the actual position of the tissue of interest compared to the position as determined in the scout scan, potentially resulting in unnecessary irradiation of tissue that is not of interest or missing part of the tissue of interest, thereby increasing the change of misdiagnosis.

In some cases, particularly in situation where the subject is not able to stay motionless (e.g. due to high stress, pain or mental issues) this problem becomes even larger, even when a subject is restrained to the subject support.

In trauma situation, stress levels of the subject may be increased already, but due to the often very urgent nature of the need for a scan (e.g. due to internal tissue damage or bleeding) and treatment, the time it takes for a scout scan already takes up valuable potential treatment time. Since radiation dose is usually considered to be less important in such cases the operator often immediately performs a full scan with an overly broad scan range either determined visually away from the subject at the control console or from memory after observing the subject close-up. While time is saved, unnecessary irradiation of tissue is very likely. Alternatively, the operator may select a too small scan range thereby missing crucial medical data necessary for diagnosis and treatment.

Therefore it seems that there is always a trade-off between scan parameter setting accuracy and speed. The present invention provides a solution that improves an accurate scan range parameter setting in a significantly faster procedure and without the need of a scout scan.

To achieve this the medical scanner 1 comprises a medical scan parameter setting device comprising a camera arrangement and a parameter provider 16.

The camera arrangement includes an optical camera 13 which has a field of view 13-1 covering at least a section of the subject support 12, preferably the whole subject support 12, and a volume above and along at least one side of the subject support 12. The camera arrangement is configured to obtain positional information and detect gestural information. Such cameras are for instance known from computer gaming controllers that remotely detect movement of a player that is then instantaneously used to control a computer game. A camera arrangement based on so-called vital signs cameras, which are known in the medical field under this and other names, also could be suitable. It is also possible to use an infra-red (heat detecting) camera instead of an optical camera. To detect the gestural information the camera arrangement comprises a gesture detector 14. This gesture detector 14 may be implemented as a software product or computer processor.

The parameter provider is configured to provide a medical scan parameter setting based on the positional information and gestural information as acquired by the optical camera.

Figure 2 schematically illustrates the operation of the medical scan parameter setting device as claimed. The camera arrangement, in this example an optical camera 13, is positioned on or near the gantry 10. It may also be positioned away from the medical imaging device, e.g. on a wall or a ceiling in the room, as long as it covers the previously mentioned field of view 13-1. The camera arrangement may also consist of a number of cameras positioned at different positions around the subject support, thereby always ensuring field-of-view 13-1 coverage from all angles that otherwise may be partially obscured by equipment, the subject 20, the operator 30 or other present medical personnel.

Before the medical scan the subject 20 to be scanned is positioned on the subject support 12. The camera 13 may preferably obtain subject positional information with respect to the subject 12, e.g. a subject outline and/or a top and bottom position of (part of) the subject with respect to the subject support 12.

An operator 30, such as a physician or radiation technologist, is positioned next to the subject support 12. To determine scan parameters instead of performing a scout scan, the operator 30 extends at least one of his hands 31-1, 31-2 such that it is in the camera field of view 13-1 and is observed by the camera 13.

The camera 13 is preferably coupled to the gesture detector 14 that is configured to analyze the image recorded by the camera 13, preferably in real-time, and is configured to recognize a human hand 31-1, 31-2 and identifies it as being the operator's hand 31-1, 31-2 (e.g. instead of the subject's hand). The position of the at least one hand 31-1, 31-2 maybe determined and stored as part of the positional information. The gesture detector 13-1 stores all positional information, comprising or consisting of one or more of the subject positional information and the hand positional information (of one or both hands) or a relative position of the at least one hand 31-1 with respect to the subject 20. The positional information may further consist of or comprise subject support positional information and/or sectional subject positional information (comprising information of a section of the subject, e.g. head, torso, arms, legs or other sections).

The gesture detector 14 is further configured to recognize a hand configuration of the at least one hand 31-1. A hand configuration may, for instance, be an extended hand or a closed fist, one or more extended fingers, a location of a top, bottom and side and orientation of the at least one hand 31-1, 31-2. Many more hand configuration would be possible, including more subtle configurations, such as bent fingers or a partially opened hand.

The gesture detector 14 is configured to determine, from all recognized hand configurations, a single gesture and/or a sequence of gestures. A gesture is defined as an intentional transition from a firsthand configuration to a second hand configuration. In contrast non-intentional transitions of hand configurations are merely considered to be 'hand movements' and should not be considered 'gestures' in light of the present invention Examples include rotating the hand 31-1, 31-2 from a vertical to a horizontal position, extending or retracting one or more fingers, moving the hand 31-1, 31-2 up and down or sideways, etc.

The gesture detector 14 is preferably coupled to the at least one camera 13 and configured to receive image data of the operator, and preferably subject and subject support, and to detect hand configurations and gestures therein.

The gesture detector 14 may use (machine) learning algorithms that will train the gesture detector 14 to improve analyzing and recognizing the gestures and variations therein between different (or even the same) operator or positions and thereby increase the change of detecting a gesture and reduce marking unintentional hand movements as gestures.

Preferably the operator 30 enters the camera field-of-view 13-1 with his at least one hand 31-1, 31-2 in a pre-defined start hand configuration, e.g. a horizontal outstretched hand, but any other start distinctive hand configuration is suitable. In such a case the gesture detector 14 recognizes the start hand configuration and will only start determining a gesture or a sequence of gestures from that moment, thereby ignoring earlier potential detection of hands or hand movements, e.g. during placing and positioning of the subject 20 on the subject support 12. A distinctive start hand configuration or gesture may also be used to indicate to the gesture detector that this hand is an operator's hand 31-1, 31-2 and that all other detected hands and movements thereof must be ignored.

Preferably the operator 30 ends the gesture with a pre-defined end hand configuration (which may be different or the same as the start hand configuration). The gesture detector 14 recognizes the end hand configuration and ignores any further changes in hand configuration before the scan or until a new start hand configuration is detected to start a new gesture or sequence of gestures). A final end hand configuration (e.g. a 'thumbs up', touching the subject 20 or subject support 12 or any other suitable hand configuration) may indicate that no further gestures will follow. Alternatively the gesture detector 14 may detect that the at least one hand 31-1 has moved outside the camera field-of-view 13-1 for a predetermined time period to indicate no more gestures will follow or other begin and end indicators may be used such as voice commands or confirmations detected by a microphone 18.

After the gesture detector 14 detected a gesture or a sequence of gestures it transmits this gestural information (comprising for instance of photographic images, treated photographic images or coded data representing the gesture) to a gesture comparer 15. The gesture comparer 15 includes or is connected to a gesture database 15-1 on which a database comprising a plurality of reference gestures is stored (again, for instance, as photographic images, treated photographic images or coded data representing the gesture). Each reference gesture is associated with a unique scan parameter. The detected gesture is compared by the gesture comparer 15 to the stored reference gestures in the gesture database 15-1 until a match is found. The gesture comparer 15 then generates gesture meaning information comprising the scan parameter associated with the reference gesture that matches the detected gesture.

In case the gesture comparer 14 fails to match the detected gesture with a reference gesture in the gesture database 15-1, the operator is informed, visually or audibly, that the gesture was not recognized. The operator then can repeat the procedure, potentially taking more care that the gesture is performed accurately and within good sight of the camera arrangement in its field-of-view 13-1, until the gesture is recognized and matched to a scan parameter such that the gesture comparer 15 can generate the gesture meaning information. The gesture comparer 15 may use (machine) learning algorithms that will train the gesture comparer 15 to improve analyzing and comparing the gestures and variations therein between different (or even the same) operator and thereby provide less non-matching results. Next, the gesture detector 15 transmits the gesture meaning information to a parameter provider 16. The parameter provider 16 receives the positional information from the camera arrangement and the gesture meaning information from the gesture comparer 15 and combines these into a scan parameter setting (or settings) that is provided to the operator 30 or directly into the control console of the medical scanning device 1.

Preferably the operator 30 acknowledges the provided scan parameter data using a confirmation (or rejection) gesture, pushing a button or a spoken acknowledgment (or rejection) to be detected by the microphone 18.

After acknowledgement of the scan parameter, further scan parameters may be entered or the scan may start. Preferably the scan starts automatically after acknowledgment of a final scan parameter, for instance using a gesture indicating the end of all scan parameters to be entered. More preferably, particularly for radiation scanners, the scan starts with a short, predetermined time delay that allows the operator 30 to relocate to a location that is shielded from the radiation. Alternatively the operator 30 manually starts the scan using the determined scan parameters using a gesture, pushing a button or by a voice command.

A scan parameter that may be particularly efficiently determined using the device and method as claimed include a scan start position and end position, defining a scan range. The scan start position may simply be determined by the position of an operator's firsthand 31-1 above the subject. A gesture indicating this to be the start position (e.g. moving the hand upside down, rotating the hand, extending one or more fingers, touching the subject 20, etc.), the scan parameter setting device sets this position as the start position. A similar procedure may be performed to set the scan end position. In a particularly efficient variation the start and end positions are determined simultaneously whereby the operator 30 extends both hands 31-1, 31-2 and the position of the first hand 31-1 is set as the scan start position and the position of the second hand 31-2 is determined as the scan end position. The operator 30 may move the hands 31-1, 31-2 closer or farther away from each other before he acknowledges the final start and end positions.

Alternatively the operator 30 may 'draw' the scan range by moving one hand 31-1, with for instance an extended finger on or above the subject 20. In a variation he may 'draw' a position of the region-of-interest on or above the subject 20 such that the scan parameter may determine the optimal scan range using anatomical information that is based on the subject (e.g. from previous scans) or general information from a database.

Other scan parameters that may be set using gesture recognition may include for instance a dose setting (taking into account the tissue to be radiated and subject characteristics); object location, such as an organ of interest location or a metallic object location (e.g. implants such as a pace maker or prostheses); subject information (such as subject gender, weight, age), which may be used in determining scan parameters or that may be stored along with the other information for reference. Further gestures may be used to start a scan (e.g. in interventional procedures when the operator 30 stays at the subject's side or with a time delay for diagnostic scans to allow the use 30 to relocate to a radiation shielded location). Similarly a scan abort signal may be gestured to immediately stop the scan, for instance if the operator 30 notices that the scan will not be performed correctly (e.g. due to subject movement), thereby avoiding unnecessary radiation dose and reducing time delays and avoiding complications during interventional procedures.

The gesture detector 14, gesture comparer 15 and parameter provider 16 may be implemented in a single or different computer processors.

The scan parameter setting device and procedure as claimed does not require a time consuming and dose-delivering scout scan, resulting in a more efficient and safer procedure that may take only moments. This is particularly important in trauma situations where the main scan may now be performed earlier and a faster diagnosis may be obtained and treatment may be started faster. Also for non-trauma situations setting scan parameters as claimed by the present invention is beneficiary, since it increases device efficiency, subject throughput and reduces radiation exposure to subjects and hospital staff (due to the absence of a scout scan).

Also due to the shorten procedural time there is less chance of voluntary or involuntary movements of the subject that may cause the scan to be less accurate or not useable, requiring rescans, with additional radiation dose and time delays. And, the procedure will be significantly less uncomfortable for the subject, since the procedure is shorter and the subject has to remain immobile for a shorter period during the procedure.

In an embodiment, the scan parameter setting device may also include a projection device 17 that may assist the operator 30 to obtain more accurate scan parameter settings and/or to obtain an even more efficient scan parameter setting procedure.

The projection device 17 may be any device capable of projecting images on the subject 20 and/or subject support 12, such as optical beamers, directional laser systems, etc.

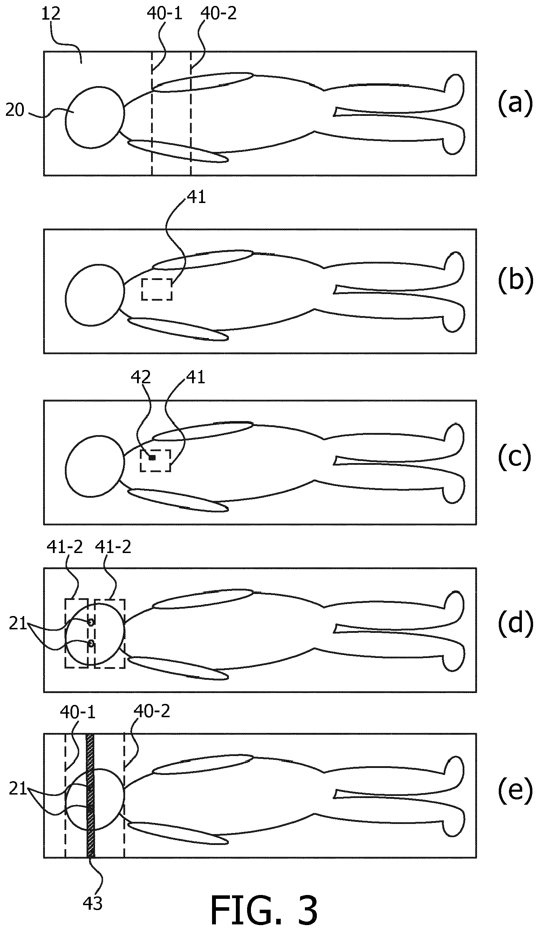

Figure 3 illustrates several, non-limiting, examples of how projections may assist the operator 30 in providing optimal scan parameter settings. The examples are also illustrative for a device without a projection device, since they also represent areas as indicated by the operator 30 even if they are not projected.

In figure 3a the operator 30 indicated a scan start position and as scan end position, in this example forming a scan area covering the subject's chest region. The projection device 18 projects lines 40-1, 40-2 on the subject 20 and subject support, whereby a first projection line 40-1 indicates the scan start position and the second projection line 40-2 indicates the scan end position as the scan position setting device determined from the operator's gestures. In practice when the subject is moved through the gantry, the radiation beam is switched on when the scan start position enters the examination area 11-3 and switches off when the scan end position enters the examination area 11-3.

In figure 3b the projection device projects a line drawing of a rectangle according to a scan area 41 that the operator 'drew' on or above the subject, in this example the scan area 41 corresponds to the heart area of the subject that will be imaged in a coronary scan.

In figure 3c the operator gestured the presence and position of an implant (in this case a pace maker) and the projection device 17 projects the position of this implant 42 in the cardiac scan area 41. The scan parameters may now be adapted such that scan parameters for image acquisition near the implant may potentially be adapted to optimize image quality in that area (e.g. reduce beam hardening effects).

In figure 3d scan parameters for a head scan with a radiation-based imager are determined. The operator 30 gestured two scan areas 41-1, 41-2 to avoid harmful radiation to the eyes 21 of the subject. The scan parameters are set such that the radiation is shut of when the eyes enter the field of view of the radiation beam. The projection device indicate the gestured areas 41-1, 41-2 on and around the subject's head. This has an additional benefit since it will be immediately visible if the subject moved his head and the eyes accidentally would enter an intended scan area 41-1, 41-2.

Figure 3e is more or less the opposite to figure 3d. In this example the operator indicated a scan start position and scan end position for a head scan (projected as projection lines 40-1, 40-2). In addition the operator 30 indicated a non-scan area 43 not to be scanned (again the eyes 21 of the subject). The projection device 17 projects the gestured non-scan area 43 onto the subject, which similarly as above, will assist the operator 30 to observe if the subject has not moved his eyes 21 outside the non-scan are 43. The scan parameters are set such that the radiation is shut off when the non-scan area 43 enters the radiation beam field-of-view.

The projection device 17 may be configured to project line images, areas, images and the like. The projection device 17 may be configured to project black and white, monochrome or color projections.

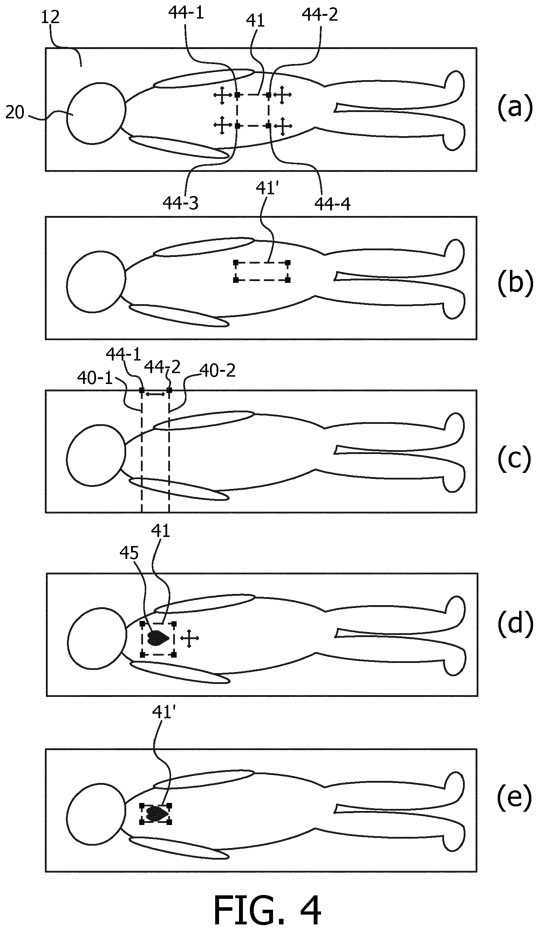

Figure 4 shows further beneficiary embodiments of the projection system and how it may assist the operator 30 to optimize hand gesture-based scan protocol settings.

In figure 4a a scan region 41 for an abdominal scan was indicated using gestures by an operator 30. The projection device 17 projects the contours of the gestured scan area on the subject's body 20. On the corners of the projected scan area 41 'drag' features 44-1, 44-2, 44-3, 44-4 are projected, in this embodiment small squares similar to those used in many computer editing programs. The operator 30 may adapt the scan are 41 by 'dragging' one or more of the drag features 44-1, 44-2, 44-3, 44-4 to make the scan area 41 larger, smaller or change its shape. Figure 4b exemplary shows how the operator 30 reconfigured the scan area 41 using the drag features 44-1, 44-2, 44-3, 44-4 to change the original square scan area 41 to a modified rectangular scan era 41'. When the operator 30 touches a projected drag feature 44-1, 44-2, 44-3, 44-4 the gesture detector 14 and gesture comparer 15 recognize that the scan area needs to be adapted and will adapt the scan area to the extent the operator 30 'drags' the drag feature 44-1, 44-2, 44-3, 44-4. The projection device 17 may update the scan area 41' in real time or after the operator 30 has stopped dragging. As such the operator 30 may set the scan parameters rather coarsely using gestures and then fine-tune the scan area 41 in a fast and simple manner to cover the area of interest, while avoiding irradiation of surrounding tissue as much as possible.

Figure 4c shows a variation of this example in which a scan start position is indicated by a first projection line 40-1 and a scan end position is indicated by a second projection line 40-2 projected by the projection device 17 across the subject 20 and subject support 12, similar to those shown in figures 4a and 4e. In this case each of the projection lines 40-1, 402 has a drag feature 44-1, 44-2 similar as in figures 4a and 4b to move one or both of the projection lines 44-1, 44-2 to enlarge or reduce the scan area. Other variations on these examples would be clear to the skilled person.

Figure 4d shows a simplified example in which previously a cardiac scan area 41 was gestured. The projection device 17 projects an outline of the gestured scan area as in figures 4a and 4b, but with the addition of anatomical information, in this case a heart (shown here extremely simplified). The heart corresponds with the known or expected position and size of the subject's 20 heart, either from a previously available scan (with the same or a different imaging modality) or determined or estimated using data from an anatomical library. The operator 30 may now reshape the scan area into an updated scan era 41 'that is much closer to the boundaries of the heart, as is shown in figure 4e. This example would of course be easily adapted for any type of anatomy of interest. The scan parameter setting device may also automatically adapt the scan area 41 to a most optimal scan area 41' based on the anatomical information. The operator may then preferably accept, adapt or reject these settings. The projected anatomy may be simplified (for instance as a contour) or detailed (for instance an actual full or segmented image of the anatomy of interest, e.g. from a previous scan or library).

Other projections that may be used to ensure optimal subject cooperation (e.g. colored projections, cartoon-like projections for children, etc.).

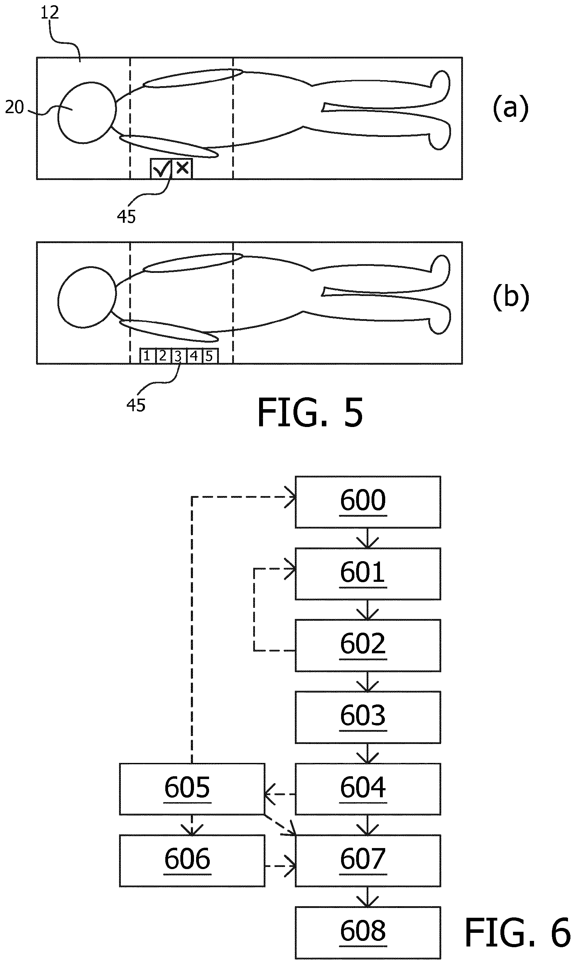

Figure 5 shows even further exemplary embodiments of how the projection device 17 may assist the gestural scan parameter setting device to enhance procedural speed.

In figure 5a the projection device 17 projects two virtual buttons 45 on the subject support 12 (or alternatively on the subject itself). In this example these are simple acknowledge (√) and rejection (X) buttons to accept or reject (projected) scan settings. Alternatively these buttons might be used to indicate the start and end of a gesture (sequence). Or they may be used to start or stop the actual scan.

In figure 5b the projection device projects even more virtual buttons 45 to give more controls to the operator 30 without the need to step away from the subject 20). In this case 5 virtual buttons are shown, but there may be more or less, for instance even a keyboard allowing the operator 30 to type in data that will then be stored with the image data or used by the parameter prover 16 to be used in determining scan parameter settings.

The exemplary projections shown in figures 3, 4 and 5, and the many variations that are possible in addition to these, allow the operator 30 to optimize the speed of determining scan parameters, but also to adapt them in a flexible and convenient manner without the need to leave the subject 20 or even to turn away to check or input data, e.g. on a computer screen. A further advantage of this is that the subject 20 is more likely to remain still when the operator 30 is at his side due to increased feeling of comfort or by reacting directly to instructions from the operator 30. Also, the operator 30 immediately notices any disturbance in the subject or his position instead compared to the known situation where he needs to step away from the subject to relocate to a control station.

Figure 6 shows an exemplary flow diagram of a method to determine scan parameter settings according to the claimed invention.

In step 600 positional information of at least one hand of an operator is obtained by the camera arrangement. This may be fully automated or triggered by a command, e.g. a pre-defined hand start position, movement of the hand into the camera field-of-view, a voice command, pressing a (virtual) button, etc.

In step 601 gestural information is obtained. Each gesture or gesture sequence may be started 601-1 with a predefined start hand position or gesture.

Steps 600 and 601 may be performed concurrently or in reverse order.

In step 602 the obtained gestural information is recognized, preferably by comparing to pre-determined gestures, e.g. gestures stored in a database.

In case it was not possible to match the obtained gestural information with a predetermined gesture the gesture must be repeated (after indicating to the operator to do so) and step 601 and 602 performed again.

In case the gesture was recognized the gesture is linked to a scan parameter setting in step 603. Preferably this is done through previously associating a gesture with a specific scan parameter and store this in the same or a different database with the predetermined gestures.

In step 604 the positional and gestural information are combined and scan parameter settings are generated based on the positional and gestural information and preferably displayed to the operator (e.g. on a monitor or by projecting on the subject and/or subject support).

Preferably the scan parameter settings are checked by the operator (step 605). In case the operator rejects the proposed scan parameters the procedure is cancelled and has to be redone from the first step. Alternatively the operator manually adapts the scan parameter settings (in step 606) to match with his intended settings.

After operator acknowledgment, or automatically, the scan parameters are transmitted to the medical imager control station used in the scan (step 607) and the scan parameters are preferably automatically set using the generated scan parameter settings.

The subject may then be scanned (step 608), automatically or after acknowledgment from the operator.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The terms "he" or "his" do not limit the claims to male persons, but were used for brevity and should be read as "he/she" or "his/her". A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. Medical scan parameter setting device for a medical imaging device for scanning a subject by an operator controlling the medical imaging device, comprising:
- a camera arrangement comprising at least one camera (13) to obtain positional information of at least part of the operator and a gesture detector (14) configured to detect gestural information from the operator;
- a parameter provider (16) configured to provide a medical scan parameter setting based on said positional information and said gestural information.

2. Device according to claim 1, wherein the positional information includes:
a position of at least one hand of the operator; and, preferably,
a position of at least part of the subject and/or of a subject support for supporting the subject during a medical scan with the medical imaging device; and, more preferably,
a relative position of the at least one hand of the operator in relation to the position of at least part of the subject and/or the subject support.

3. Device according to claim 2, wherein the gestural information includes at least one gesture of the at least one hand of the operator, particularly a sequence of gestures of the at least one hand of the operator.

4. Device according to any of the previous claims, wherein the gesture detector (14) is configured to detect at least one gesture from image data obtained by the at least one camera (13).

5. Device according to any of the previous claims further comprising a gesture comparer (15), that is configured generate gestural information by comparing the detected at least one gesture to a predefined gesture, preferably from a gesture library (15-1), that has a predefined meaning relating to the medical scan, wherein the gestural information comprises the predefined meaning.

6. Device according to claim 5, wherein the predefined meaning consists of a meaning selected from the group of:
- start position;
- end position;
- scan speed;
- dose setting;
- object location, such as an organ of interest location or a metallic object location;
- subject information, such as subject gender, weight, age;
- scan start signal; and/or
- scan abort signal.

7. Device according to any of the previous claims, wherein the parameter provider is configured to generate the medical scan parameter by combining gestural information and positional information and transmitting said combined information to a medical scan parameter.

8. Device according to any of the previous claims wherein the medical scan parameter consists of at least one of a scan parameter selected from the group of:
- start position;
- end position;
- scan speed;
- minimum scan dose;
- maximum scan dose; and/or
- object location, such as an organ of interest location or a metallic object location.

9. Device according to any of the previous claims, further comprising:
- a projection device configured to, when in use with a subject present, project information relating to the determined scan parameters on or near the subject, for instance:
- a scan parameter preview, such as a scan range preview;
- virtual tools, such as scan parameter modification tools; and/or
- scan control tools.

10. Device according to claim 9, wherein the projection device is further configured to project additional subject data on or near the subject, such as
- general subject data, such as subject gender, weight, age;
- anatomical information; and/or
- subject relevant medical history information, such as previous scan images, processed information from previous scan images, list of medical conditions and/or presence and/or type of implants.

11. Medical imaging device comprising a medical scan setting device according to any of the previous claims.

12. Method for setting a medical scan parameter for a medical imaging device for scanning a subject by an operator controlling the medical imaging device, comprising the steps of:
- obtaining (600) positional information of at least one hand of the operator; and, preferably, a position of at least part of the subject and/or of a subject support; and, more preferably, a relative position of the at least one hand of the operator in relation to the position of at least part of the subject and/or the subject support;
- obtaining (601) gestural information that includes a gesture of the at least one hand of the operator, particularly a sequence of gestures of the at least one hand of the operator;
- provide (604) a medical scan parameter setting based on said positional information and said gestural information.

13. Method according to claim 12, wherein the medical scan parameter is provided by combining gestural information and positional information and relating said combined information to a medical scan parameter.

14. Method according to claim 12 or 13, wherein a predetermined gesture or sequence of gestures is compared (602) to a predefined gesture, preferably from a gesture library, that has a predefined meaning relating to the medical scan and wherein this predefined meaning is translated into a medical scan parameter.

15. Computer program product that is configured, when running, to execute any of the steps of claims 12 to 14.
